**Europäisches Patentamt**

(19) **European Patent Office**

**Office européen des brevets**

(11) Veröffentlichungsnummer: **0 053 241**
**B1**

(12) EUROPÄISCHE PATENTSCHRIFT

(45) Veröffentlichungstag der Patentschrift:
**12.09.84**

(21) Anmeldenummer: **81108022.5**

(22) Anmeldetag: **07.10.81**

(51) Int. Cl.³: **C 07 C 69/82,** C 07 C 67/39,
C 07 C 67/58, B 01 J 23/92

(54) **Verfahren zur Gewinnung und Wiederverwendung von Schwermetalloxidationskatalysator aus Rückständen im Witten-DMT-Prozess.**

(30) Priorität: **02.12.80 DE 3045332**

(43) Veröffentlichungstag der Anmeldung:
**09.06.82 Patentblatt 82/23**

(45) Bekanntmachung des Hinweises auf die Patenterteilung:
**12.09.84 Patentblatt 84/37**

(84) Benannte Vertragsstaaten:
**DE FR IT NL**

(56) Entgegenhaltungen:
**DE - A - 2 249 605**

(73) Patentinhaber: **DYNAMIT NOBEL
AKTIENGESELLSCHAFT, Postfach 1209,
D-5210 Troisdorf, Bez. Köln (DE)**

(72) Erfinder: **Bünger, Heinrich, Dr., Fliederweg 11,
D-5200 Siegburg-Kaldauen (DE)**
Erfinder: **Cordes, Rudolf, Dr., Kasseler Weg 3,
D-5216 Niederkassel (DE)**
Erfinder: **Hoffmann, Gerhart, Dr., Porzerstrasse 1,
D-5216 Niederkassel (DE)**

**Beschreibung**

Die Erfindung betrifft ein Verfahren zur Gewinnung und Wiederverwendung von Schwermetalloxidationskatalysator aus Rückständen im Witten-DMT-Prozess der im Oberbegriff des Anspruches 1 bezeichneten Art.

Dimethylterephthalat (DMT) wird in zahlreichen grosstechnischen Anlagen nach dem Witten-DMT-Verfahren (vgl. DE-PS 1 041 945) hergestellt. Durch Umsetzung mehrfunktioneller Alkohole mit DMT werden Polyester gewonnen. Solche — auch als gesättigte Polyester bezeichneten hochmolekularen Verbindungen — werden unter anderem zu Fasern, Fäden, Filmen oder Formteilen verarbeitet.

Nach dem Witten-DMT-Verfahren wird ein Gemisch von p-Xylol (PX) und p-Toluylsäuremethylester (PTE) in der flüssigen Phase in Abwesenheit von Lösungsmitteln und von Halogenverbindungen bei einem Druck von etwa 4 bis 8 bar und einer Temperatur von etwa 140 bis 170°C mit Luftsauerstoff in Gegenwart von gelösten Schwermetalloxidationskatalysatoren oxidiert, z.B. in Gegenwart eines Gemisches von Kobalt- und Mangan-Verbindungen (vgl. DE-PS 2 010 137); daneben sind für das Witten-DMT-Verfahren noch andere Katalysatoren untersucht worden, die neben Kobalt und/oder Mangan noch Nickel, Cer oder Beryllium enthalten (vgl. H. Bünger: Compendium 78/79, Ergänzungsband der Zeitschrift Erdöl und Kohle, Erdgas, Petrochemie, S. 417-436).

Im Anschluss an die Oxidationsstufe wird das erhaltene Reaktionsgemisch, das überwiegend aus Monomethylterephthalat (MMT) und aus p-Toluylsäure (PTS) besteht, mit Methanol bei einem Druck von etwa 20 bis 25 bar und einer Temperatur von etwa 250 bis 280°C verestert. Das Veresterungsprodukt wird destillativ aufgetrennt in eine PTE-Fraktion, eine DMT-Fraktion und hochsiedenden teerartigen Rückstand. Die PTE-Fraktion wird in die Oxidation zurückgeführt. Der hochsiedende teerartige Destillationsrückstand enthält unter anderem sämtliche Bestandteile des Katalysatorsystems.

Es entspricht einem technischen Bedürfnis, die Oxidationskatalysatoren aus dem teerartigen Destillationsrückstand zurückzugewinnen und erneut zur Oxidation von PX und PTE einzusetzen.

Es wurde bereits vorgeschlagen, den Oxidationskatalysator aus hochsiedendem Destillationsrückstand, der bei der DMT-Herstellung nach dem Witten-DMT-Verfahren anfällt, durch Flüssig-Flüssig-Extraktion zu gewinnen und den katalysatorhaltigen Extrakt in die Oxidationsstufe zurückzuführen. Als Extraktionsmittel wurden z.B. Wasser oder Gemische aus Wasser und wasserlöslichen Carbonsäuren vorgeschlagen (vgl. japanische Patentanmeldung 67/42 997). Eine solche Flüssig-Flüssig-Extraktion liefert jedoch Extrakte mit inkonstanter und oft zu niedriger katalytischer Aktivität und Selektivität; deshalb wurden verschiedene zusätzliche Massnahmen vorgeschlagen, um Aktivitäts- und Selektivitätseinbussen zu vermeiden (vgl. DE-OS 2 525 135, DE-AS 2 531 106).

Auch diese Verfahren können noch nicht voll befriedigen. Durch unvollständige Extraktion entstehen Katalysatorverluste, und es treten Betriebsstörungen durch Emulsionsbildung auf. Ausserdem sind die Katalysatorkonzentrationen in den Extrakten niedrig, so dass die Extrakte vor erneutem Einsatz in die Oxidationsstufe aufkonzentriert werden müssen. Zum Aufkonzentrieren sind spezielle Vorrichtungen notwendig, die erhebliche Investitions- und Betriebskosten verursachen. Aus den aufkonzentrierten Extrakten kristallisieren während des Lagerns verschiedene Substanzen aus; dadurch wird die Handhabung der Extrakte erschwert.

Weiterhin enthalten die Extrakte Trimellitsäure und Trimellitsäuremonomethylester; diese können die katalytische Aktivität und Selektivität beeinträchtigen (vgl. DE-AS 2 923 681).

Bei den vorgenannten Verfahren wird neben den Katalysatorbestandteilen auch Eisen aus dem Destillationsrückstand extrahiert, das aus dem Anlagenwerkstoff stammt. Dieses Eisen wird bei ständiger Rückführung des Extrakts in die Oxidation umso stärker im Katalysatorkreislauf angereichert, je vollständiger die Extraktion der Schwermetalle ist. Eine stärkere Eisenanreicherung ist jedoch unerwünscht, weil auch hierdurch die Selektivität der Oxidation beeinträchtigt wird. Ausserdem enthält der nach den vorgenannten Verfahren extrahierte Rückstand stets Wasser, welches bei einer anschliessenden weiteren Verwendung des Rückstands stören kann.

Die Aufgabe der Erfindung ergibt sich demgemäss aus dem geschilderten Stand der Technik und den nachfolgend aufgeführten Vorteilen und Wirkungen der Erfindung: Erhöhung des Wiedergewinnungsgrades an Schwermetalloxidationskatalysator, Verringerung der Extraktionsmittelmenge und Erhalt eines Extraktes mit erhöhter Katalysatorkonzentration, verringerter Gehalt an Trimellitsäure, Trimellitsäuremonomethylester und beim Lagern auskristallisierender Substanzen im Extrakt, Vermeidung einer unzulässigen Anreicherung von Eisen im Katalysatorkreislauf trotz erhöhten Schwermetallextraktionsgrades sowie Erhalt eines extrahierten Rückstandes mit niedrigem Wassergehalt.

Diese Aufgabe wird erfindungsgemäss in einem Verfahren der eingangs bezeichneten Art durch die in dem Kennzeichen des Anspruches 1 angegebenen Massnahmen gelöst.

Die erfindungsgemässe Extraktion, bei der Rückstand und Extraktionsmittel im Gegenstrom geführt werden, kann aus zwei oder mehr Extraktionsstufen bestehen. Mit zunehmender Anzahl der Extraktionsstufen kann die auf die Rückstandsmenge bezogene Extraktionsmittelmenge verringert werden. Vorteilhaft sind zwei bis sechs Extraktionsstufen.

Nach dem Stand der Technik werden Destillationsrückstand und Extraktionsmittel während einer Zeit von 10 bis 240 Minuten miteinander vermischt. Erfindungsgemäss kann eine für die Extraktionsaufgabe ausreichende Vermischung von Rückstand mit Extraktionsmittel auch innerhalb von 0,1 bis 400 Sekunden, bevorzugt innerhalb von 0,1 bis 10 Sekunden erfolgen. Bei einer besonders bevorzugten Ausführungsform der Erfindung werden Rückstand und Extraktionsmittel in der ersten Extraktionsstufe während einer Zeit von 10 bis 240 Minuten miteinander vermischt, in allen folgenden Extraktionsstufen während einer Zeit von 0,1 bis 10 Sekunden miteinander vermischt. Diese kurzzeitige Vermischung kann zum Beispiel erfolgen, indem Rückstand und Extraktionsmittel gleichzeitig in die Saugseite einer Kreiselpumpe gespeist und gemeinsam in den Absetzbehälter gepumpt werden.

Unterhalb des Siedebeginns der wässrigen Phase wird der Extraktionsgrad mit steigender Absetztemperatur höher, gleichzeitig wird der Wassergehalt des extrahierten Rückstands niedriger. Es ist deshalb vorteilhaft, die Temperaturen in den Absetzbehältern nicht niedriger als 70°C einzustellen; vorzuziehen ist, die Absetztemperaturen nicht niedriger als 15°C unterhalb des Siedebeginns der wässrigen Phase einzustellen, und es ist besonders vorteilhaft, die Absetztemperaturen nicht niedriger als 5°C unterhalb des Siedebeginns der wässrigen Phase einzustellen. Falls die wässrigen Phasen in den Absetzbehältern sieden, kann der Druck unerwünscht ansteigen, und die Phasentrennung kann verlangsamt werden; deshalb ist es vorteilhaft, die Absetztemperaturen so einzustellen, dass in den Absetzbehältern nicht mehr als 40 Gew.-% der wässrigen Phase abdestillieren; es ist vorzuziehen, die Absetztemperaturen nicht oberhalb des Siedebeginns der wässrigen Phase einzustellen, und es ist besonders vorteilhaft, die Absetztemperaturen nicht höher als 1°C unterhalb des Siedebeginns der wässrigen Phase einzustellen.

Es können jedoch auch höhere oder niedrigere Temperaturen eingestellt werden, wenn z.B. durch grössere Absetzbehälter, durch erhöhte Extraktionsstufenzahl, durch erhöhte Extraktionsmittelmenge den veränderten Verhältnissen Rechnung getragen wird. Die Temperaturen in den Mischern werden zweckmässig auf den etwa gleichen Werten gehalten wie in den Absetzern.

Unter erhöhtem Druck ist die Siedekurve der wässrigen Phase nach höheren Temperaturen verschoben, deshalb können bei erhöhtem Druck und erhöhter Temperatur kleinere Absetzbehälter verwendet werden; die Absetztemperatur soll jedoch auch bei erhöhtem Druck nicht oberhalb von 160°C liegen. Es ist allerdings vorzuziehen, die Phasentrennung unter Normaldruck durchzuführen.

Mit sinkendem Mengenverhältnis von wässriger zu organischer Phase steigt die Katalysatorkonzentration im wässrigen Extrakt, dadurch entstehen Vorteile, weil geringere Extraktmengen gehandhabt werden müssen und weil bei ausreichend hohen Katalysatorkonzentrationen die sonst notwendige Eindampfung oder eine andere Aufkonzentrierung des Extraktes entfallen kann. Mit sinkendem Mengenverhältnis von wässriger zu organischer Phase geht jedoch im allgemeinen der Extraktionsgrad zurück.

Es ist ein besonderer Vorteil der erfindungsgemässen Arbeitsweise, dass niedrigere Mengenverhältnisse von wässriger zu organischer Phase angewandt werden können, ohne dass der Extraktionsgrad erheblich verringert wird. Deshalb wird bei der erfindungsgemässen Arbeitsweise im allgemeinen ein Mengenverhältnis von wässriger zu organischer Phase eingestellt, das nicht höher als 9 : 10, vorzuziehen ist ein Mengenverhältnis von 8 : 10 oder darunter, insbesondere 5 : 10. Bei sehr niedrigem Mengenverhältnis von wässriger zu organischer Phase kann es allerdings notwendig werden, die Anzahl der Extraktionsstufen zu erhöhen. Deshalb wird man im allgemeinen ein Mengenverhältnis von 1 : 10 nicht unterschreiten, vorzuziehen ist ein Mengenverhältnis nicht niedriger als 2 : 10, besonders vorzuziehen ein Mengenverhältnis von 3 : 10 oder darüber. Selbstverständlich können aber auch höhere Mengenverhältnisse eingestellt werden als 9 : 10; dadurch wird zwar der Extrakt stärker verdünnt, doch bleiben die anderen, durch die Erfindung erreichbaren Vorteile erhalten.

Die zweckmässige Verweilzeit der organischen Phase in den Absetzbehältern hängt vom Katalysatorgehalt des Rückstands, von der Anzahl der Extraktionsstufen, von der Absetztemperatur und dem Mengenverhältnis von wässriger zu organischer Phase ab. Bei zu kurzer Verweilzeit ist die Phasentrennung unzureichend, eine zu lange Verweilzeit führt zu einem erhöhten Volumenbedarf für die Absetzbehälter. Die Verweilzeit kann im zweiten und jedem folgenden Absetzbehälter geringer sein als im jeweils vorhergehenden Absetzbehälter. Diejenige Verweilzeit, die unter den jeweiligen Bedingungen zu ausreichender Phasentrennung führt, kann durch wenige Versuche ermittelt werden.

Der DMT-Gehalt des zu extrahierenden Destillationsrückstandes kann mehr als 10 Gew.-% DMT betragen; er kann auch niedriger sein, z.B. infolge einer Nachbehandlung, etwa nach der DE-PS 2 310 824 oder der DE-PS 2 427 875. Falls der erfindungsgemässen Extraktion Nachbehandlungen vorhergehen, die die Viskosität des Rückstands wesentlich erhöhen, sind Massnahmen notwendig, um eine ausreichende Dispergiergeschwindigkeit der wässrigen und der organischen Phase in den Mischern zu erreichen. Eine ausreichende Dispergiergeschwindigkeit kann z.B. durch Einstellen geeigneter Temperaturen in den Mischern erreicht werden oder durch Verdünnen der organischen Phase mit einem niedriger viskosen, mit dem Rückstand mischbaren und mit Wasser wenig oder nicht mischbaren Verdünnungsmittel, z.B. mit p-Xylol, Mischxylol oder aromatischen Estern. Dabei sind Verdünnungsmittel vorzuziehen, deren Dichte bei den Extraktionstemperaturen höher ist als die Dichte von Wasser und Benzoesäuremethylester ist besonders vorteilhaft.

Selbstverständlich kann auch zuerst die erfindungsgemässe Katalysatorrückgewinnung und daran anschliessend eine Rückstandsnachbehandlung durchgeführt werden.

Für das erfindungsgemässe Verfahren können diejenigen Extraktionsmittel verwendet werden, die bereits vorgeschlagen wurden für die Extraktion von Oxidationskatalysator aus hochsiedendem Destillationsrückstand, der bei der DMT-Herstellung nach dem Witten-Verfahren anfällt; vorzuziehen sind wässrige Lösungen niederer aliphatischer Carbonsäuren, und besonders vorzuziehen ist das essig- und ameisensäurehaltige Reaktionsabwasser, das bei der Oxidation von PX und PTE entsteht. Dieses Reaktionsabwasser fällt als Brüdenkondensat an; es kann ohne weitere Behandlung als Extraktionsmittel verwendet werden, es kann aber auch nach Abtrennung derjenigen Bestandteile, die leichter als Wasser sieden, als Extraktionsmittel verwendet werden.

Der erfindungsgemäss gewonnene Extrakt kann mit Vorteil direkt in die Oxidationsstufe des Wittener-DMT-Verfahrens wieder eingesetzt werden, ohne eingedampft oder auf andere Art aufkonzentriert zu werden und ohne dass unlösliche Kobaltverbindungen oder organische Stoffe daraus abgetrennt werden müssen.

Falls im erfindungsgemäss gewonnenen Extrakt eine unerwünschte Anreicherung von Eisen festgestellt wird, kann der Eisengehalt durch Filtration des Extraktes auf einen zulässigen Wert reduziert werden; dabei ist es vorteilhaft, den Extrakt bei erhöhter Temperatur durch ein grobkörniges Filterhilfsmittel zu filtrieren, wobei Aktivkohle besonders vorteilhaft ist.

Die mit der Erfindung erreichbaren Vorteile bestehen insbesondere darin, dass trotz erheblich reduzierter Extraktionsmittelmenge ein wesentlich höherer Katalysatorwiedergewinnungsgrad erreicht wird, dass ein Extrakt mit höherer Katalysatorkonzentration gewonnen wird, dass dieser Extrakt ohne eingedampft und ohne auf andere Art aufkonzentriert zu werden in der Oxidationsstufe des Wittener-DMT-Verfahrens verwendet werden kann, dass der Extrakt keine störenden Mengen beim Lagern auskristallisierender Substanzen enthält, dass der Gehalt des Extraktes an Trimellitsäure und Trimellitsäuremonomethylester, bezogen auf seinen Gehalt an Oxidationskatalysator, verringert ist, dass der extrahierte Rückstand weniger Wasser enthält und dass trotz erhöhten Katalysatorrückgewinnungsgrades keine unzulässige Anreicherung von Eisen im Katalysatorkreislauf stattfindet.

Die folgenden Beispiele 1 und 2 dienen der Erläuterung der erfindungsgemässen Arbeitsweise und der erfindungsgemäss realisierten Vorteile, wobei die Vergleichsbeispiele 2 und 4 den Stand der Technik, von dem die Erfindung ausgeht, illustrieren.

*Beispiel 1*

Extrahiert wurde der gesamte Destillationsrückstand, der in der Rohesterdestillation einer industriellen Anlage zur DMT-Herstellung nach dem Witten-Verfahren anfiel.

In dieser Anlage wurden PX und PTE gemeinsam in flüssiger Phase bei 5 - 7 bar Überdruck und 150 bis 170°C Reaktionstemperatur in einer Kaskade aus drei Oxidatoren kontinuierlich oxidiert; als Oxidationskatalysator diente eine Lösung von Kobaltacetat und Manganacetat in dem Reaktionsabwasser, das bei dieser Oxidation entsteht und als Brüdenkondensat anfällt; dieses Reaktionsabwasser enthält durchschnittlich 2,5 Gew.-% Essigsäure, 1,5 Gew.-% Ameisensäure, 6,0 Gew.-% Methanol und 0,8 Gew.-% Formaldehyd. Die Katalysatorlösung wurde kontinuierlich in den ersten Oxidator der Kaskade eingespeist, so dass sich im Oxidationsprodukt eine stationäre Konzentration von 90 ppm Kobalt und 9 ppm Mangan einstellte. Das Oxidationsprodukt enthielt neben dem genannten Katalysator und den entstandenen Carbonsäuren nicht umgesetztes Einsatzprodukt und verschiedene Zwischen- und Nebenprodukte. Dieses Oxidationsprodukt wurde bei Temperaturen von etwa 250°C und Drücken zwischen 20 und 30 bar kontinuierlich mit Methanol verestert. Das Veresterungsprodukt wurde kontinuierlich durch Vakuumdestillation aufgetrennt; diejenigen Bestandteile, die höher als DMT siedeten, wurden unter Vakuum einer thermischen Nachbehandlung unterworfen, wobei weiteres DMT aus den hochsiedenden Bestandteilen abdestillierte.

Der so erhaltene Destillationsrückstand enthielt 0,40 Gew.-% Kobalt und 400 ppm Mangan. Dieser Rückstand wurde kontinuierlich einem mit Rührer versehenen Mischbehälter zugeführt. In diesem Mischbehälter wurde der Rückstand mit der wässrigen Phase gemischt, die aus einem «Absetzbehälter B» genannten Behälter ablief. Aus dem Mischbehälter wurde kontinuierlich durch ein Bodenventil eine Emulsion in einen «Absetzbehälter A» genannten Behälter abgelassen und dort in eine wässrige und eine rückstandhaltige organische Phase getrennt. Beide Phasen wurden kontinuierlich aus dem Absetzbehälter A abgezogen. Die organische Phase wurde mit einer Kreiselpumpe aus dem Absetzbehälter A in den Absetzbehälter B gepumpt; in das Saugrohr dieser Kreiselpumpe wurde gleichzeitig mit der organischen Phase das oben beschriebene essig- und ameisensäurehaltige Reaktionsabwasser eingespeist; beide wurden gemeinsam in den Absetzbehälter B gepumpt. Auf 3,75 Gewichtsteile Rückstand wurde ein Gewichtsteil Extraktionsmittel eingesetzt. Die Temperaturen im Mischbehälter und in den Absetzbehältern wurden auf 94°C gehalten. Die mittlere Verweilzeit im Mischbehälter betrug 4 Stunden, die mittlere Verweilzeit in der Kreiselpumpe betrug 1,2 Sekunden, die mittlere Verweilzeit der geschlossenen organischen Phase im Absetzbehälter A betrug 20 Stunden, die mittlere Verweilzeit der geschlossenen organischen Phase im Absetzbehälter B betrug 10 Stunden.

Aus dem Absetzbehälter B wurde extrahierter Rückstand abgezogen, der 8 ppm Kobalt und weniger als 1 ppm Mangan enthielt. Daraus ergibt sich ein Wiedergewinnungsgrad von 99,8%.

Der Wassergehalt dieses extrahierten Rückstands, bestimmt nach der Karl-Fischer-Methode, betrug 0,35 Gew.-%.

Die aus dem Absetzbehälter A ablaufende wässrige Phase enthielt 1,5 Gew.-% Kobalt, 0,15 Gew.-% Mangan, insgesamt 0,74 Gew.-% Trimellitsäure und Trimellitsäuremonomethylester sowie 230 ppm Eisen. Diese Lösung wurde 240 Stunden bei 80°C gelagert, dabei kristallisierten pro Liter Lösung 1,2 g Festsubstanzen aus.

*Beispiel 2*

Es wurde gearbeitet wie in Beispiel 1, mit der einzigen Ausnahme, dass die aus dem Absetzbehälter A ablaufende Lösung durch grobkörnige Aktivkohle abfiltriert wurde. Es wurden die gleichen Ergebnisse erhalten wie in Beispiel 1, mit dem einzigen Unterschied, dass der Eisengehalt der so filtrierten Lösung 15 ppm betrug. Diese Lösung wurde in den ersten Oxidator der in Beispiel 1 beschriebenen Oxidatorkaskade zurückgeführt; die Oxidation wurde ohne Schwierigkeiten mit dieser Katalysatorlösung betrieben.

*Beispiel 3* (Vergleichsbeispiel)

Der in Beispiel 1 beschriebene Rückstand wurde mit dem in Beispiel 1 beschriebenen Extraktionsmittel in dem in Beispiel 1 beschriebenen Mischbehälter gemischt; die entstehende Emulsion wurde durch das Bodenventil in den Absetzbehälter A abgelassen und dort in eine wässrige und eine rückstandshaltige organische Phase getrennt. auf ein Gewichtsteil Rückstand wurde ein Gewichtsteil Extraktionsmittel eingesetzt. Die Temperaturen im Mischbehälter und im Absetzbehälter wurden auf 94°C gehalten. Die mittlere Verweilzeit im Mischbehälter betrug 2,7 Stunden, die mittlere Verweilzeit der geschlossenen organischen Phase im Absetzbehälter betrug 30 Stunden.

Aus dem Absetzbehälter wurde extrahierter Rückstand abgezogen, der 320 ppm Kobalt und 32 ppm Mangan enthielt; daraus ergibt sich ein Wiedergewinnungsgrad von 92%.

Der Wassergehalt des extrahierten Rückstands betrug 3,2 Gew.-%.

Die aus dem Absetzbehälter ablaufende wässrige Phase enthielt 0,37 Gew.-% Kobalt und 370 ppm Mangan. Sie wurde eingedampft und enthielt nach dem Eindampfen 1,5 Gew.-% Kobalt, 0,15 Gew.-% Mangan, insgesamt 1,6 Gew.-% Trimellitsäure und Trimellitsäuremonomethylester sowie 270 ppm Eisen. Die eingedampfte Lösung wurde 240 Stunden bei 80°C gelagert; während des Lagerns kristallisierten pro Liter eingedampfter Lösung 32 g Festsubstanzen aus, die vor Einsatz der Lösung in die Oxidationsstufe abgetrennt werden mussten.

*Beispiel 4* (Vergleichsbeispiel)

Der in Beispiel 1 beschriebene Rückstand wurde mit dem in Beispiel 1 beschriebenen Extraktionsmittel unter den gleichen Bedingungen wie in Beispiel 3 extrahiert, mit dem einzigen Unterschied, dass auf zwei Gewichtsteile Rückstand ein Gewichtsteil Extraktionsmittel eingesetzt wurde. Jetzt wurde nur ein Wiedergewinnungsgrad von 67,6% erhalten, und der Wassergehalt des extrahierten Rückstands betrug 4,1 Gew.-%. Die aus dem Absetzbehälter ablaufende wässrige Phase enthielt 0,50 Gew.-% Kobalt und 0,05 Gew.-% Mangan. Nach dem Eindampfen enthielt sie 1,5 Gew.-% Kobalt, 0,15 Gew.-% Mangan, insgesamt 1,5 Gew.-% Trimellitsäure und Trimellitsäuremonomethylester sowie 220 ppm Eisen. Die eingedampfte Lösung wurde 240 Stunden bei 80°C gelagert, dabei kristallisierten pro Liter eingedampfter Lösung 26 g Festsubstanzen aus, die vor Einsatz der Lösung in die Oxidationsstufe abgetrennt werden mussten.

**Patentansprüche**

1. Verfahren zur Gewinnung und Wiederverwendung von Schwermetalloxidationskatalysator aus Rückständen im Witten-DMT-Prozess durch Extraktion unter Vermischen und Absitzenlassen bei 70 bis 160°C mit Wasser oder verdünnten wässrigen Lösungen wasserlöslicher, niedermalekularer, aliphatischer Monocarbonsäuren und Rückführung des Extraktes der hochsiedenden Destillationsrückstände, die bei der Oxidation von p-Xylol und p-Toluylsäuremethylester enthaltenden Gemischen in flüssiger Phase mit Sauerstoff oder einem Sauerstoff enthaltenden Gas bei erhöhtem Druck und erhöhter Temperatur in Gegenwart von gelöstem Schwermetalloxidationskatalysator, anschliessenden Veresterung des Oxidationsprodukts mit Methanol bei erhöhtem Druck und erhöhter Temperatur und destillativen Auftrennung des Veresterungsprodukts in eine Roh-DMT-Fraktion, eine p-Toluylsäuremethylesterreiche Fraktion und einen hochsiedenden Destillationsrückstand anfallen, dadurch gekennzeichnet dass hochsiedender Destillationsrückstand und Extraktionsmittel durch zwei oder mehr Extraktionsstufen im Gegenstrom in einem Mengenverhältnis von 1 : 0,9 bis 1 : 0,1, vorzugsweise 1 : 0,5 bis 1 : 0,3, geführt werden.

2. Verfahren nach Anspruch 1, dadurch gekennzeichnet, dass der Destillationsrückstand und das Extraktionsmittel bei Temperaturen zwischen 85 bis 100°C innerhalb 0,1 bis 400 Sekunden miteinander vermischt werden.

3. Verfahren nach Anspruch 1 und 2, dadurch gekennzeichnet, dass der katalysatorhaltige Extrakt vor der Rückführung in die Oxidation filtriert wird.

4. Verfahren nach Anspruch 1 bis 3, dadurch gekennzeichnet, dass die organische Phase vor der Extraktion mit einem niedriger viskosen Verdünnungsmittel versetzt wird, das mit Rückstand mischbar und mit Wasser wenig oder nicht mischbar ist.

5. Verfahren nach Anspruch 4, dadurch gekennzeichnet, dass als niedriger viskoses Verdünnungsmittel Benzoesäuremethylester eingesetzt wird.

**Claims**

1. Process for the recovery and reuse of heavy metal oxidation catalyst from residues in the Witten-DMT process by extraction, while undergoing mixing and allowing to settle out at 70 to 160°C, using water or diluted aqueous solutions of water-soluble, low molecular aliphatic monocarboxylic acids, and return of the extract of the high boiling distillation residues which accumulate in the oxidation of p-xylene and p-toluic acid methyl ester-containing mixture in the liquid phase with oxygen or an oxygen-containing gas at elevated pressure and elevated temperature in the presence of dissolved heavy metal oxidation catalyst, then esterification of the oxidation product with methanol at elevated pressure and elevated temperature and distillative separation off of the esterification product into a crude DMT fraction, a p-toluic acid methyl ester-rich fraction and a high boiling distillation residue, characterised in that high boiling distillation residue and extraction medium are led through two or more extraction steps in counter-current in a mass ratio 1 : 0.9 to 1 : 0.1, preferably 1 : 0.5 to 1 : 0.3.

2. Process according to claim 1, characterised in that the distillation residue and the extraction medium are mixed with one another at temperatures between 85 to 100°C, within 0.1 to 400 seconds.

3. Process according to claim 1 and 2, characterised in that the catalyst containing extract is filtered off before the return to the oxidation.

4. Process according to claim 1 to 3, characterised in that the organic phase is treated before the extraction with a lower viscosity diluent which is miscible with residue and slightly miscible or not at all miscible with water.

5. Process according to claim 5, characterised in that benzoic acid methyl ester is employed as diluent of lower viscosity.

## Revendications

1. Procédé de récupération et de réutilisation d'un catalyseur d'oxydation, à base de métaux lourds, à partir de résidus obtenus dans le procédé Witten de production de téréphtalate de diméthyle par extraction avec mélangeage à 70°C à 160°C avec de l'eau ou avec des solutions aqueuses diluées d'acides monocarboxyliques aliphatiques à bas poids moléculaire, hydrosoluble, puis dépôt, et recyclage de l'extrait des résidus de distillation à point élevé d'ébullition, que l'on obtient lors de l'oxydation en phase liquide de mélanges contenant du p-xylène et de l'ester méthylique de l'acide p-toluylique avec de l'oxygène ou avec un gaz contenant de l'oxygène à pression élevée et température élevée en présence d'un catalyseur d'oxydation à base de métal lourd dissous, puis estérification du produit d'oxydation par du méthanol sous pression élevée et à température élevée et séparation par distillation du produit d'estérification en une fraction de téréphtalate de diméthyle brut, une fraction riche en ester méthylique de l'acide p-toluylique et un résidu de distillation à point élevé d'ébullition, caractérisé en ce qu'on soumet le résidu de distillation à point élevé d'ébullition et l'agent d'extraction à deux ou plusieurs étapes d'extraction à contre-courant selon un rapport de masse compris entre 1 : 0,9 et 1 : 0,1, et de préférence entre 1 : 0,5 et 1 : 0,3.

2. Procédé selon la revendication 1, caractérisé en ce qu'on mélange le résidu de distillation et l'agent d'extraction l'un avec l'autre à des températures comprises entre 85 et 100°C en l'espace de 0,1 à 400 secondes.

3. Procédé selon les revendications 1 et 2, caractérisé en ce que l'on filtre l'extrait contenant du catalyseur avant de le recycler vers l'oxydation.

4. Procédé selon les revendications 1 à 3, caractérisé en ce que, avant l'extraction, on ajoute à la phase organique un diluant peu visqueux, qui est miscible avec le résidu et est peu ou pas miscible à l'eau.

5. Procédé selon la revendication 4, caractérisé en ce qu'on utilise comme diluant peu visqueux de l'ester méthylique de l'acide benzoïque (benzoate de méthyle).